Europäisches Patentamt

European Patent Office

Office européen des brevets

(11) Publication number: **0 093 540**
**B1**

(12) **EUROPEAN PATENT SPECIFICATION**

(45) Date of publication of patent specification: **14.09.88**

(51) Int. Cl.⁴: **C 07 C 37/07, C 07 C 39/08**

(21) Application number: **83302225.4**

(22) Date of filing: **19.04.83**

(54) Production of Hydroquinone.

(30) Priority: **29.04.82 US 372874**

(43) Date of publication of application:
**09.11.83 Bulletin 83/45**

(45) Publication of the grant of the patent:
**14.09.88 Bulletin 88/37**

(84) Designated Contracting States:
**BE DE FR GB IT NL**

(56) References cited:
**EP-A-0 070 665**
**EP-A-0 084 448**
**US-A-3 213 114**
**US-A-3 794 668**
**US-A-3 859 365**
**US-A-3 987 068**
**TETRAHEDRON LETTERS, no. 10, 1977, pages 821-824, Pergamon Press, GB; D. BONDON et al.: "Nouvelle méthode d'aromatisation de cyclohexenones contenues dans des systèmes polycycliques"**

(73) Proprietor: **Sun Refining and Marketing Company**
**1801 Market Street**
**Philadelphia, PA 19103-1699 (US)**

(72) Inventor: **Chao-Yang, Hsu**
**615 Farnum Road**
**Media, PA 19063 (US)**
Inventor: **Lyons, James E.**
**211 Copper Drive**
**Wallingford, PA 19086 (US)**

(74) Representative: **Lewin, John Harvey et al**
**Elkington and Fife High Holborn House 52/54 High Holborn**
**London WC1V 6SH (GB)**

(56) References cited:
**JOURNAL OF THE AMERICAN CHEMICAL SOCIETY, vol. 61, no. 8, 5th August 1939, pages 2230-2234, US; M. CALVIN: "Homogeneous catalytic hydrogenation"**

## Description

This invention relates to the production of hydroquinone.

It is known that phenol can be oxidised to p-benzoquinone in the presence of copper catalysts; see for example US—PS 3,987,068. Improvements to these copper-catalyzed oxidations of phenol to p-benzoquinone have been disclosed and claimed in EP—A—0070665 and EP—A—0084448.

Heretofore, p-benzoquinone has been converted to hydroquinone by the use of various catalysts. Thus, for example, Sabatier passed quinone vapours mixed with hydrogen over a reduced copper catalyst (e.g., a heterogeneous vapour-phase system) to obtain hydroquinone (Comptes rendus Ac. Sc. vol. 146, p. 457, 1908 and vol. 172 p. 733, 1921), but in this process the catalyst has a very limited life. R. Cornubert and J. Phelisse, *Compt. Rend. 229,* 460 (1949) disclose use of Raney nickel to convert quinone to hydroquinone. US-PS 2,495,521 discloses nickel, cobalt, or copper-catalyzed heterogeneous vapour phase hydrogenation of benzoquinone in the presence of steam. Popova et al., *Chem. Abs.* 53,275 (1959) disclose hydrogenation of benzoquinone with nickel, platinum and palladium — calcium carbonate. M. Calvin, J. Am. Chem. Soc. *61* 2230 (1939) discloses use of a copper-quinoline complex derived from cuprous acetate to promote hydrogenation of purified quinone in quinoline solution, but this process is limited in that, at most, only one-half mole of hydrogen is taken up per mole of the Copper I — quinoline complex and thus the hydrogenation is very inefficient and perhaps not catalytic. Also of interest is the disclosure of Yananaka et al., *Bull. Inst. Phys. Chem. Research* (Tokyo) *14,* 31 1935 which reduces quinones to oxy compounds with copper and $Al_2O_3$-promoted nickel.

We have now found that a good yield of hydroquinone is readily obtained when the p-benzoquinone in a reaction mass obtained by oxidation of phenol with a copper catalyst is hydrogenated directly, *i.e.,* without isolation, and without any additional catalyst. It is quite obvious that such a "one-pot" process provides a highly efficient means to obtain hydroquinone which is a commercial chemical much used in the photographic industry and as an inhibitor used in the stabilisation of rubber compounds.

Thus, the present invention provides a process for making hydroquinone from phenol, comprising (a) reacting phenol with oxygen in the presence of a copper catalyst to form p-benzoquinone, (b) removing unreacted oxygen, and (c) in the same reaction mass without additional catalyst or separation of the p-benzoquinone, reacting the p-benzoquinone with hydrogen in a homogeneous liquid phase reaction to form hydroquinone.

The oxidation of phenol to p-benzoquinone with a copper catalyst is, as indicated, well known in the art and any of such processes may be used.

Preferably, a process such a that disclosed in US—PS 3,987,068 will be used. Most preferably, the hydrogenation step of this invention will be used with the processes disclosed in (a) EP—A— 0070665 where a divalent copper catalyst promoted with an alkali metal base is used (molar ratio of base to copper being no greater than about 2.0) and may also be further enhanced with water in an amount less than about 10% by volume of the reaction solution, and (b) European Patent Application No. 83300210.8 where a monovalent copper catalyst promoted with water is used (preferably 1 to 4 moles of water per mole of phenol).

The hydrogenation step in the process will be carried out in a homogeneous, liquid phase simply by first removing oxygen from the system and then pressurizing in hydrogen generally to a pressure of 34 to 345 bar gauge, preferably 69 to 207 bar gauge (about 500 to about 5000 psig, preferably about 1000 to about 3000 psig) and effecting the hydrogenation at a temperature generally from about 100° to about 200°C, preferably from about 125° to about 175°C.

In order to further exemplify the process, the following Examples are given:

### Example 1

500 mmole of phenol are oxidised in 350 ml of actonitrile containing 35 mmole of $CuCl_2$. An oxygen-containing gas (39% $O_2$, 61% $N_2$) is continuously sparged through the mixture at 65°C and 52 bar (750 psi) at 500 ml/hr. After four hours the reaction mixture contains four mmole of phenol, 263 mmole of benzoquinone and six mmole of p-chlorophenol (99% conv., 53% selectivity). The oxygen is flushed from the system and hydrogen gas is admitted to a pressure of 207 bar (3,000 psi). The mixture is heated for fifteen hours at 175°C to give 135 mmoles of hydroquinone, 2 mmoles of unreacted p-benoquinone, 17 mmoles of catechol, 6 mmoles of p-chlorophenol, 17 mmoles of phenol and a large amount of quinhydrone (a 1:1 complex of hydroquinone and p-benzoquinone). Yield calculations are difficult because of the low accuracy of the quinhydrone analysis, but a yield of up to about 71% is believed to be achieved.

### Example 2

In an experiment similar to Example 1, except that the $CuCl_2$ catalyst is promoted with LiOH (one mole per mole of $CuCl_2$), selectivity to p-benzoquinone is about 70% at 99% conversion. The reaction mixture is hydrogenated by pressurizing 138 bar (2000 psi) of hydrogen on it at 175°C to give a yield of hydroquinone about the same as in Example 1.

### Example 3

Similar results are obtained when the process of Example 1 is carried out with a CuCl catalyst promoted with 500 mmole of water.

## Claims

1. A process for making hydroquinone from phenol, comprising (a) reacting phenol with oxygen in the presence of a copper catalyst to form, p-benzoquinone, (b) removing unreacted oxygen, and (c) in the same reaction mass without additional catalyst or separation of the p-benzoquinone, reacting the p-benzoquinone with hydrogen in a homogeneous liquid phase reaction to form hydroquinone.

2. A process as claimed in claim 1, wherein the copper catalyst used in the oxidation of phenol is divalent and is promoted with an alkali metal base.

3. A process as claimed in claim 1, whrrein the copper catalyst used in the oxidation of phenol is a monovalent copper catalyst promoted with water.

4. A process as claimed in any of claims 1 to 3, wherein the hydrogenation is carried out under a hydrogen pressure of from 34 to 345 bar gauge and at a temperature of from 100 to 200°C.

## Patentansprüche

1. Verfahren zur Herstellung von Hydrochinon aus Phenol, bei dem (a) Phenol mit Sauerstoff in Gegenwart eines Kupfer-Katalysators unter Bildung von p-Benzochinon umgesetzt wird, (b) nichtumgesetzter Sauerstoff entfernt wird und (c) in der gleichen Reaktionsmasse ohne zusätzlichen Katalysator oder Abtrennung des p-Benzochinons das p-Benzochinon mit Wasserstoff in einer homogenen Flüssigphasenreaktion unter Bildung von Hydrochinon umgesetzt wird.

2. Verfahren nach Anspruch 1, bei dem der in der Oxidation von Phenol eingesetzte Kupfer-Katalysator zweiwertig ist und eine Alkalimetallbase als Promotor enthält.

3. Verfahren nach Anspruch 1, bei dem der in der Oxidation von Phenol eingesetzte Kupfer-Katalysator ein einwertiger Kupfer-Katalysator ist, der Wasser als Promoter enthält.

4. Verfahren nach einem der Ansprüche 1 bis 3, bei dem die Hydrierung unter einem Wasserstoffdruck von 34 bis 345 bar über Atmosphärendruck und bei einer Temperatur von 100 bis 200°C durchgeführt wird.

## Revendications

1. Procédé de fabrication d'hydroquinone à partir de phénol, qui comprend (a) le réaction du phénol avec de l'oxygène en présence d'un catalyseur au cuivre pour former de la p-benzoquinone, (b) l'élimination de l'oxygène n'ayant pas réagi, et (c), dans la même masse réactionelle, sans apport de catalyseur ou séparation de la p-benzoquinone, réaction de celle-ci avec de l'hydrogène, en phase liquide homogène, pour former l'hydroquinone.

2. Procédé selon la revendication 1, dans lequel le catalyseur au cuivre, utilisé pour l'oxydation du phénol, est divalent, et est activé avec une base de métal alcalin.

3. Procédé selon la revendication 1, dans lequel le catalyseur au cuivre utilisé pour l'oxydation du phénol est un catalyseur au cuivre monovalent, activé avec de l'eau.

4. Procédé selon une des revendications 1 à 3, dans lequel l'hydrogénation est réalisée sous pression d'hydrogène de 34 à 345 bars et à une température de 100° à 200°C.